# FASCICULE DE BREVET EUROPEEN

(11) **EP 1 336 382 B1**
(45) Date de publication et mention de la délivrance du brevet: **07.09.2005**
(21) Numéro de dépôt: 03290251.2
(22) Date de dépôt: 03.02.2003
(51) Int. Cl.: A61B 17/70, A61B 17/80

(54) **Système permettant de solidariser une barre ou analogue avec au moins une vertèbre**
System zum Verbinden eines Stabes oder dergleichen mit einem Wirbelknochen
System for for connecting a spinal rod or the like with at least one vertebrae

(30) Priorité: 13.02.2002 FR 0201749
(43) Date de publication de la demande: 20.08.2003
(73) Titulaire: Razian, Hassan, 94240 l'Hay les Roses (FR)
(72) Inventeur: Razian, Hassan, 94240 l'Hay les Roses (FR)
(74) Mandataire: Flavenot, Bernard

(56) Documents cités:
- EP-A- 1 121 903
- WO-A-02/09605
- FR-A- 2 761 876

## Description

La présente invention concerne les systèmes pour solidariser une barre ou analogue avec au moins une vertèbre, qui trouvent une application particulièrement avantageuse pour le maintien d'au moins deux vertèbres, consécutives ou non, l'une par rapport à l'autre en vue de la réalisation d'une arthrodèse rachidienne, par exemple chez un être humain dans le but de supprimer par exemple la cause des douleurs générées par disque intervertébral endommagé ou une fracture de vertèbre, ou pour traiter une scoliose ou une cyphose ou pour éviter le risque de complications paralytiques liées à cette fracture.

Les praticiens dans le domaine de la chirurgie ostéo-rachidienne utilisent notamment un système constitué par des plaques dans lesquelles sont pratiqués un orifice et une lumière, et des vis comportant chacune une tige à filetage osseux terminée par une tête d'épaulement, ces vis étant aptes à coopérer avec les plaques en se vissant dans l'os des corps vertébraux pour que ces plaques soient emprisonnées en appui entre les vertèbres et les têtes d'épaulement des vis.

Ce système d'ostéosynthèse rachidienne est intéressant mais peut dans certains cas présenter des inconvénients, notamment par le fait qu'il nécessite l'implantation de vis dans les corps vertébraux, implantation qui peut se révéler risquée et difficile compte tenu de la configuration de ces corps vertébraux et/ou entraîner de graves complications.

Pour pallier ces inconvénients, certains praticiens utilisent des systèmes essentiellement constitués de crochets aptes à coopérer avec les lames, pédicules ou apophyses des vertèbres et de barres qui sont solidarisées par tous moyens à ces crochets. Ces systèmes éliminent les inconvénients mentionnés ci-dessus, mais en présentent d'autres, essentiellement de deux types : la difficulté de positionner les crochets sur les lames, pédicules ou apophyses en même temps que les solidariser avec une barre, et leur tenue dans le temps, sur ces apophyses.

Un tel système est par exemple décrit dans le US-A-5 733 284. Il est cependant visible sur les figures annexées et confirmé dans la description que le système selon ce document comporte une ou plusieurs entretoises nécessitant l'utilisation de vis qui pénètrent dans la partie osseuse de la vertèbre, ainsi que de crochets, cerclages ou analogues qui s'appuient sur les apophyses au risque de les abîmer.

La présente invention a donc pour but de réaliser un système pour solidariser une barre ou analogue avec au moins une vertèbre, qui pallie au moins en partie les inconvénients mentionnés ci-dessus des systèmes d'ostéosynthèse rachidienne selon l'art antérieur.

Plus précisément, la présente invention a pour objet un système permettant de solidariser une barre ou analogue avec au moins une vertèbre en accord avec le contenu de la revendication 1 annexée dont le préambule tient compte du contenu de l'art antérieur, et plus particulièrement du document américain mentionné ci-dessus.

D'autres caractéristiques et avantages de l'invention apparaîtront au cours de la description suivante donnée en regard des dessins annexés à titre illustratif mais nullement limitatif, dans lesquels :
Les figures 1, 3 et 4 représentent, vus en perspective cavalière, trois modes de réalisation de l'élément "embase" du système selon l'invention permettant de solidariser une barre avec au moins une vertèbre,
La figure 2 représente une vue de face un exemple de moyens pour fixer la barre avec l'embase, et
Les figures 5 et 6 représentent, à titre d'application, trois modes de réalisation possibles de l'embase montés en coopération avec respectivement trois vertèbres consécutives en vue postérieure, la figure 5 représentant trois embases positionnées sur trois vertèbres avant leur fixation avec deux barres, la figure 6 représentant ces trois embases solidarisées avec les deux barres.

Il est bien précisé que, sur les figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

Le Demandeur tient aussi à préciser que les figures représentent plusieurs modes de réalisation de l'objet selon l'invention, mais qu'il peut exister d'autres modes de réalisation qui répondent à la définition de cette invention.

Il précise en outre que, lorsque, selon la définition de l'invention, l'objet de l'invention comporte "au moins un" élément ayant une fonction donnée, le mode de réalisation décrit peut comporter plusieurs de ces éléments.

Il précise aussi que, si les modes de réalisation de l'objet selon l'invention tel qu'illustré comportent plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

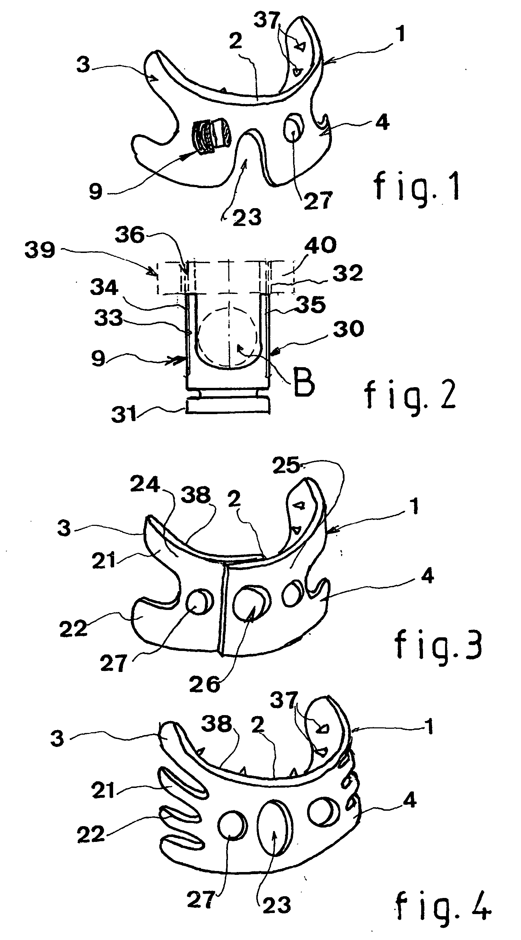

Les figures 1, 3 et 4 représentent trois modes de réalisation d'un système permettant de solidariser une barre B ou analogue avec au moins une vertèbre V, qui comporte une embase 1 en forme sensiblement de "U" comprenant un fond 2 et deux branches latérales 3, 4 solidaires de deux bords opposés de ce fond. L'embase 1 est conformée pour que son fond 2 vienne en recouvrement de la partie postérieure de la vertèbre V en entourant au moins partiellement son apophyse épineuse Aₑ et que ses deux branches latérales 3, 4 viennent se positionner de part et d'autre de la vertèbre V en entourant au moins partiellement respectivement ses deux apophyses transverses At1, At2, et de façon que ces deux branches latérales 3, 4 prennent appui par pincement latéralement sur au moins l'une des éléments suivants : les deux faces 5, 6 les plus éloignées l'une de l'autre des deux pédicules 7, 8 et sur les apophyses transverses de la vertèbre V.

Le système comporte en outre des moyens 9 pour fixer la barre B avec l'embase 1. Un exemple de ces moyens 9 est décrit ci-après.

Comme illustré sur les figures 1, 3 et 4, les deux branches latérales 3, 4 de l'embase 1 peuvent prendre plusieurs formes, mais sont avantageusement constituées chacune d'au moins deux pattes 21, 22 solidaires du fond 2, les deux pattes 21, 22 étant espacées l'une de l'autre de façon qu'elles soient aptes à se positionner de part et d'autre d'une apophyse transverse Aₜ₁, Aₜ₂.

Comme mentionné ci-avant, le fond 2 de l'embase 1 est conformé de façon qu'il vienne en recouvrement de la partie postérieure de la vertèbre V en entourant au moins partiellement l'apophyse épineuse Aₑ de cette vertèbre. Aussi, dans une réalisation possible comme illustré sur la figure 1, pour réaliser cette fonction, ce fond 2 comporte-t-il une lumière ouverte 23 pour le passage de l'apophyse épineuse Aₑ, tandis que, sur les deux figures 3 et 4, cette lumière est fermée.

Dans une réalisation préférentielle, les branches latérales 3, 4 sont réalisées en un matériau élastiquement déformable pour constituer les branches d'une pince élastique qui peuvent alors parfaitement se plaquer en force contre les deux faces 5, 6 des pédicules et donc assurer le positionnement de l'embase sur la vertèbre V. Ce matériau élastiquement déformable peut être par exemple du titane ou analogue, en sachant que l'embase 1 peut être réalisée d'une seule pièce dans ce même matériau, ou d'un assemblage de plusieurs pièces, par exemple de tiges formées et de plaques.

Dans une réalisation possible qui permet par exemple d'ajuster la distance séparant les deux branches latérales à la largeur de la vertèbre V, l'embase 1 est constituée, comme illustré sur la figure 3, de deux demi-U 24, 25 et de moyens 26 pour accoupler les deux demi-U par leur fond.

Ces moyens 26 pour accoupler les deux demi-U par leur fond peuvent être constitués de différentes façons. Cependant, dans une réalisation avantageuse, ils peuvent être constitués de deux trous réalisés chacun dans le fond d'un demi-U 24, 25, le premier trou, celui qui est réalisé dans le fond du demi-U qui est situé vers l'intérieur de la forme en U de l'embase reconstituée étant taraudé, tandis que le second trou est de forme générale oblongue, et d'une vis à tête conique apte à se visser dans le premier trou.

Dans ce cas, la tête conique vient en appui contre un côté du second trou oblong pour translater, au fur et à mesure du vissage, le fond du demi-U dans lequel est réalisé ce second trou, sur le fond du demi-U dans lequel est réalisé le premier trou. Ce dernier moyen n'est bien entendu qu'un moyen avantageux qui permet une amplitude de déplacement relatif des deux demi-U l'un vers l'autre qui peut être suffisante pour cette application.

Comme mentionné ci-avant, le système comporte en outre des moyens 9 pour fixer la barre B avec l'embase 1.

Ces moyens 9 comportent, dans une réalisation préférentielle, un orifice 27 réalisé dans l'embase 1, une pièce oblongue 30 dont une première extrémité 31 est sertie, avantageusement en rotation, dans l'orifice 27, sa seconde extrémité 32 étant conformée en cavalier 33 à deux bras latéraux 34, 35, ce cavalier étant apte à recevoir la barre B entre ses deux bras, figure 2, et des moyens 36 pour solidariser la barre B avec ce cavalier 33. De préférence, l'orifice 27 est un orifice oblong.

Avec cette réalisation décrite ci-dessus, comme la pièce 30 peut, d'une part être pivotée puisqu'elle est sertie en rotation dans l'orifice 27, et d'autre part translatée le long de cet orifice puisqu'il est oblong, il est possible de positionner de façon relativement précise le cavalier 33 par rapport à une barre B et donc d'introduire cette barre entre les deux bras 34, 35.

Dans une réalisation avantageuse, mais non exclusive, les moyens 36 pour solidariser la barre B avec le cavalier 33 sont constitués par exemple par des moyens 39 pour rapprocher les deux bras latéraux 34, 35 du cavalier 33 en vue de pincer la barre B. Ces moyens 39 pour rapprocher les deux bras 34, 35 sont avantageusement constitués par exemple par un écrou 40 à filetage conique ou analogue apte à se visser sur les faces extérieures des deux bras 34, 35. Ainsi, lors du vissage de l'écrou 40, les deux bras se rapprochent l'un de l'autre et enserrent la barre B, la solidarisant ainsi fermement avec l'embase 1.

Pour mieux assujettir l'embase 1 avec la vertèbre V, il est avantageux que le système comporte en outre des picots 37 tapissant la paroi intérieure 38 de l'embase 1.

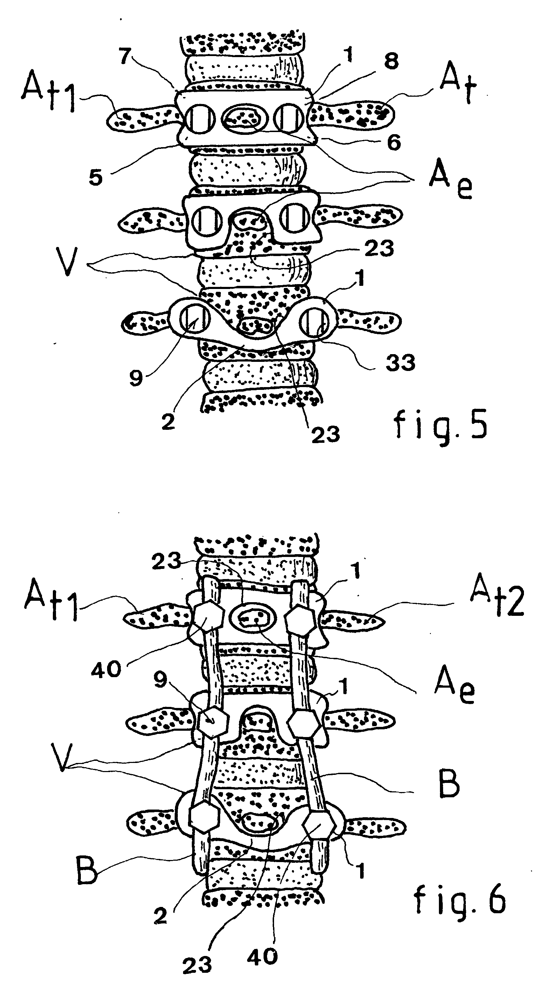

Le système selon l'invention, par référence aux figures 5 et 6, s'utilise de la façon suivante :
Les figures 5 et 6 représentent à titre d'application, trois modes de réalisation possibles de l'embase 1 montés en coopération avec respectivement trois vertèbres consécutives en vue postérieure, la figure 5 représentant trois embases positionnées sur trois vertèbres avant leur fixation avec deux barres, la figure 6 représentant ces trois embases solidarisées avec deux barres B.

Pour arriver à cette configuration, le praticien commence par choisir les embases qui ont une taille et une configuration qui leur permettent de recouvrir et de pincer comme décrit ci-avant les vertèbres V qui doivent être soumises à une arthrodèse.

Il place alors les trois embases 1 respectivement sur les trois vertèbres V, figures 5, puis prépare les deux barres B en leur donnant approximativement la forme qui peut leur permettre de passer entre les bras des cavaliers 33. Il présente ensuite ces deux barres en regard des cavaliers qu'il déplace éventuellement en translation et/ou en rotation pour que les barres puissent être introduites entre les deux bras 34, 35 des cavaliers.

Il visse enfin un écrou 40 sur les extrémités 32 de chaque cavalier de façon à enserrer fermement les barres et ainsi les maintenir parfaitement solidaires des embases.

De ce fait, puisque ces embases sont elles-mêmes solidaires des trois vertèbres, ces trois vertèbres sont parfaitement maintenues les unes par rapport aux autres et l'arthrodèse peut ainsi se réaliser, et ce, sans avoir eu de difficulté pour l'implantation des embases car chacune se stabilise immédiatement par rapport à la vertèbre sur laquelle elle a été placée, et sans avoir dû percer les corps vertébraux.

## Revendications

1. Système permettant de solidariser une barre (B) ou analogue avec au moins une vertèbre (V), comportant au moins une embase (1) agencée pour venir en recouvrement de la partie postérieure de la vertèbre en entourant au moins partiellement l'apophyse épineuse (Aₑ) de ladite vertèbre (V) et des moyens (9) pour fixer ladite barre (B) avec ladite embase (1), **caractérisé par le fait que** ladite embase (1) présente une forme sensiblement de "U" comportant un fond (2) et deux branches latérales (3, 4) solidaires de deux bords opposés du fond, ledit fond (2) venant en recouvrement de la partie postérieure de la vertèbre en entourant au moins partiellement l'apophyse épineuse (Aₑ) et les deux branches latérales (3, 4) venant se positionner de part et d'autre de la vertèbre en entourant au moins partiellement respectivement ses deux apophyses transverses (At1, At2), et de façon que ces deux branches latérales (3, 4) prennent appui latéralement en pincement, pour solidariser ladite embase à la vertèbre sans ajout d'éléments annexes, sur au moins l'un des éléments suivants: les deux faces (5, 6) les plus éloignées l'une de l'autre des deux pédicules (7, 8) et les apophyses transverses de la vertèbre (V).

2. Système selon la revendication 1, **caractérisé par le fait qu'**au moins une branche latérale (3, 4) est constituée d'au moins deux pattes (21, 22) solidaires du fond (2), les deux dites pattes (21, 22) étant espacées l'une de l'autre de façon qu'elles soient aptes à se positionner de part et d'autre d'une apophyse transverse (Aₜ₁, Aₜ₂)

3. Système selon l'une des revendications 1 et 2, **caractérisé par le fait que** ledit fond (2) comporte une lumière (23) pour le passage de l'apophyse épineuse (Ae).

4. Système selon l'une des revendications 1 à 3, **caractérisé par le fait qu'**au moins les branches latérales (3, 4) sont réalisées en matériau élastiquement déformable.

5. Système selon l'une des revendications 1 à 4, **caractérisé par le fait que** l'embase (1) est constituée de deux demi-U (24, 25) et de moyens (26) pour accoupler les deux demi-U par leur fond.

6. Système selon l'une des revendications 1 à 5, **caractérisé par le fait que** les moyens (9) pour fixer la barre (B) avec l'embase comportent un orifice (27) réalisé dans l'embase (1), une pièce oblongue (30) dont une première extrémité (31) est sertie en rotation dans l'orifice (27), sa seconde extrémité (32) étant conformée en cavalier (33) à deux bras latéraux (34, 35), ledit cavalier étant apte à recevoir la barre (B) entre ses deux bras, et des moyens (36) pour solidariser la barre (B) avec le cavalier (33).

7. Système selon la revendication 6, **caractérisé par le fait que** les moyens (36) pour solidariser la barre (B) avec le cavalier (33) sont constitués par des moyens (39) pour pincer les deux bras latéraux (34, 35) sur la barre (B).

8. Système selon l'une des revendications 6 et 7, **caractérisé par le fait que** l'orifice (27) est un orifice oblong.

9. Système selon l'une des revendications 1 à 8, **caractérisé par le fait qu'**il comporte des picots (37) tapissant la paroi intérieure (38) de l'embase (1).

## Patentansprüche

1. System, das ermöglicht, einen Stab (B) oder dergleichen mit wenigstens einem Wirbel (V) fest zu verbinden, das wenigstens eine Befestigungsfläche (1), die so beschaffen ist, dass sie den hinteren Teil des Wirbels abdecken kann, indem sie wenigstens teilweise den dornartigen Fortsatz (Aₑ) des Wirbels (F) umgibt, und Mittel (9), um den Stab (B) an der Befestigungsfläche (1) zu befestigen, umfasst, **dadurch gekennzeichnet, dass** die Befestigungsfläche (1) im Wesentlichen "U"-förmig ist und einen Boden (2) sowie zwei seitliche Schenkel (3, 4), die mit zwei gegenüberliegenden Rändern des Bodens fest verbunden sind, umfasst, wobei der Boden (2) den hinteren Teil des Wirbels abdeckt, indem er wenigstens teilweise den dornartigen Knochenfortsatz (Aₑ) umgibt, und die beiden seitlichen Schenkel (3, 4) beiderseits des Wirbels angeordnet werden, indem sie wenigstens teilweise jeweils dessen zwei transversale Knochenfortsätze (At1, At2) umgeben, und derart, dass sich diese zwei seitlichen Schenkel (3, 4) seitlich durch Einstechen, um die Befestigungsfläche am Wirbel ohne Hinzufügung zusätzlicher Elemente fest anzubringen, an wenigstens einem der folgenden Elemente abstützen: den beiden am weitesten voneinander entfernten Flächen (5, 6) der zwei Pedunkuli (7, 8) und den transversalen Knochenfortsätzen der Wirbelsäule (V).

2. System nach Anspruch 1, **dadurch gekennzeichnet, dass** wenigstens ein seitlicher Schenkel (3, 4) aus wenigstens zwei mit dem Boden (2) fest verbundenen Ansätzen (21, 22) gebildet ist, wobei diese zwei Ansätze (21, 22) voneinander so beabstandet sind, dass sie sich beiderseits eines transversalen Knochenfortsatzes (Aₜ₁, Aₜ₂) befinden.

3. System nach einem der Ansprüche 1 und 2, **dadurch gekennzeichnet, dass** der Boden (2) ein Langloch (23) für den Durchgang des dornartigen Knochenfortsatzes (Ae) aufweist.

4. System nach einem der Ansprüche 1 bis 3, **dadurch gekennzeichnet, dass** wenigstens einer der seitlichen Schenkel (3, 4) aus einem elastisch verformbaren Material verwirklicht ist.

5. System nach einem der Ansprüche 1 bis 4, **dadurch gekennzeichnet, dass** die Befestigungsfläche (1) aus zwei Halb-Us (24, 25) und aus Mitteln (26) zum Koppeln der zwei Halb-Us durch ihren Boden gebildet ist.

6. System nach einem der Ansprüche 1 bis 5, **dadurch gekennzeichnet, dass** die Mittel (9) zum Befestigen des Stabs (B) an der Befestigungsfläche eine in der Befestigungsfläche (1) verwirklichte Öffnung (27), ein lang gestrecktes Teil (30), wovon ein erstes Ende (31) drehbar in die Öffnung (27) eingesetzt ist und ein zweites Ende (32) als Reiter (33) mit zwei Seitenarmen (34, 35) ausgebildet ist, wobei der Reiter zwischen seinen zwei Armen den Stab (B) aufnehmen kann, und Mittel (36), um den Stab (B) mit dem Reiter (33) fest zu verbinden, umfassen.

7. System nach Anspruch 6, **dadurch gekennzeichnet, dass** die Mittel (36) zum Befestigen des Stabs (B) an dem Reiter (33) durch Mittel (39) gebildet sind, die die zwei seitlichen Arme (34, 35) in den Stab (B) stechen.

8. System nach einem der Ansprüche 6 und 7, **dadurch gekennzeichnet, dass** die Öffnung (27) eine längliche Öffnung ist.

9. System nach einem der Ansprüche 1 bis 8, **dadurch gekennzeichnet, dass** es Stacheln (37) aufweist, mit denen die Innenwand (38) der Befestigungsfläche (1) besetzt ist.

## Claims

1. A system for securing a bar (B) or the like to at least one vertebra (V), the system comprising at least one baseplate (1) arranged to cover the posterior portion of the vertebra surrounding the spinus process (Aₑ) of said vertebra (V) at least in part, and means (9) for fastening said bar (B) to said baseplate (1), **the system being characterized by the fact that** said baseplate (1) is substantially U-shaped, comprising a bottom (2) and two side limbs (3, 4) secured to two opposite edges of the bottom, said bottom (2) overlying the posterior portion of the vertebra by surrounding the spinus process (Aₑ) at least in part, and the two side limbs (3, 4) being positioned on either side of the vertebra by surrounding respective ones of its two transverse processes (Aₜ₁, Aₜ₂) at least in part and in such a manner that the two side limbs (3, 4) act to secure said baseplate to the vertebra without the addition of auxiliary elements by bearing laterally to clamp against at least one of the following elements: the two furthest-apart faces (5, 6) of the two pedicles (7, 8) and the transverse processes of the vertebra (V).

2. A system according to claim 1, **characterized by** the fact that at least one side limb (3, 4) is constituted by at least two tabs (21, 22) secured to the bottom (2), said two tabs (21, 22) being spaced apart from each other in such a manner as to be suitable for taking up positions on either side of a transverse process (Aₜ₁, Aₜ₂).

3. A system according to claim 1 or claim 2, **characterized by** the fact that said bottom (2) includes an opening (23) to pass the spinus process (Aₑ).

4. A system according to any one of claims 1 to 3, **characterized by** the fact that at least one of the side limbs (3, 4) is made of an elastically deformable material.

5. A system according to any one of claims 1 to 4, **characterized by** the fact that the baseplate (1) is constituted by two half U-shapes (24, 25) and by means (26) for coupling the two half U-shapes together via their bottoms.

6. A system according to any one of claims 1 to 5, **characterized by** the fact that the means (9) for fastening the bar (B) to the baseplate comprise an orifice (27) made in the baseplate (1), an oblong part (30) having a first end (31) rotatably crimped in the orifice (27), its second end (32) being shaped as a fork (33) having two lateral prongs (34, 35), said fork being suitable for receiving the bar (B) between its two prongs, and means (36) for securing the bar (B) to the fork (33).

7. A system according to claim 6, **characterized by** the fact that the means (36) for securing the bar (B) to the fork (33) are constituted by means (39) for clamping the two lateral prongs (34, 35) against the bar (B).

8. A system according to claim 6 or claim 7, **characterized by** the fact that the orifice (27) is an oblong orifice.

9. A system according to any one of claims 1 to 8, **characterized by** the fact that it includes spikes (37) covering the inside wall (38) of the baseplate (1).
